# EUROPEAN PATENT APPLICATION

(11) **EP 4 219 539 A1**
(43) Date of publication of application: **02.08.2023**
(21) Application number: 20954772.8
(22) Date of filing: 28.09.2020
(51) Int. Cl.: C07K 14/605, A61K 38/26, A61P 1/16, A61P 3/06, A61P 9/10

(54) **GLP-1R/GCGR DUAL-TARGET AGONIST PEPTIDE DERIVATIVES FOR TREATMENT OF VIRAL HEPATITIS-RELATED HEPATIC FIBROSIS**

(71) Applicant: Shenzhen Turier Biotech Co., Ltd., Shenzhen, Guangdong 518055 (CN)
(72) Inventor: LIU, Qi, Shenzhen Guangdong 518055 (CN); ZHANG, Binzhi, Shenzhen Guangdong 518055 (CN)
(74) Representative: SSM Sandmair
(86) International application number: PCT/CN2020/118542
(87) International publication number: WO 2022/061924

(57) **Abstract**

The present invention relates to a use of polypeptide compounds having dual agonist effect on glucagon-like peptide-1 receptor (GLP-1R) and glucagon receptor (GCGR). The polypeptide compounds are characterized by high enzymolysis stability, high potency and no adverse reaction, and capable of substantially improving hepatic fibrosis caused by hepatitis B virus (HBV) and hepatitis C virus (HCV) and severity of fibrotic conditions accompanied with liver diseases. The dual target agonist polypeptide derivatives are capable of preventing or treating hepatic fibrosis diseases associated with viral hepatitis.

## Description

### Field of the Invention

The present invention belongs to the field of biochemical technology, and particularly relates to GLP-1R/GCGR dual target agonist polypeptide derivatives. The present invention further relates to uses of the GLP-1R/GCGR dual target agonist polypeptide derivatives in prevention and/or treatment of hepatic fibrosis associated with viral hepatitis.

### Background of the Invention

Hepatic fibrosis is a pathological process caused by the unbalanced deposition of Extracellular Matrix (ECM) due to the continuous synthesis and degradation of ECM during tissue repair response against the persistent hepatic injury, and it is a dynamic process related to complex cellular and molecular mechanisms. Hepatic fibrosis is a major pathological feature of chronic liver diseases and also the main intermediate link before the further development into cirrhosis. The repair response to injury causes many changes to the ECM microenvironment, during which the quiescent hepatic stellate cells are activated, leading to changes in the forms and functions of the hepatic stellate cells, particularly, the abnormal synthesis and degradation of the ECM (Petitclerc L, Sebastiani G, Gilbert G, Cloutier G, Tang A. Liver fibrosis: Review of current imaging and MRI quantification techniques. J Magn Reson Imaging. 2017,45(5): 1276-1295.). An imbalance in ECM synthesis and degradation leads to the massive accumulation of the ECM and thus the formation of fibrosis. The occurrence of fibrosis further increases the activation of hepatic stellate cells, which aggravates fibrosis continually. The hepatic fibrosis and cirrhosis are mostly induced by the following factors: chronic HBV or HCV infection, alcohol abuse and non-alcoholic steatohepatitis (NASH) and the like (He L, Yuan H, Liang J, Hong J, Qu C. Expression of hepatic stellate cell activation-related genes in HBV-, HCV-, and nonalcoholic fatty liver disease-associated fibrosis. PLoS One. 2020;15(5):e0233702.).

Hepatic fibrosis associated with NASH is a disease induced by nonalcoholic fatty liver disease (NAFLD). In recent years, NAFLD has become the most common chronic liver disease affecting approximately 30% of the global adult population. The etiology of NAFLD is widely recognized to be related to obesity and Type I diabetes, and NAFLD is regarded as a manifestation of metabolic syndrome in the liver. Liver pathology is characterized by persistent low-grade inflammation and adipose tissue degeneration and remodeling caused by excessive lipid deposition. NAFLD can be divided into nonalcoholic simple steatosis (SS), nonalcoholic steatohepatitis (NASH), and NASH-related hepatic fibrosis and cirrhosis from the mild to severe lesion and fibrosis (The Relation Between Adiponectin and Liver Fibrosis of Nonalcoholic Fatty Liver and Viral Hepatitis, Zhai Wei, Liu Bei; School of Medical Technology, Xi'an Medical College).

Different from the pathogenesis of hepatic fibrosis related to NASH, the hepatic fibrosis related to viral hepatitis is related to HBV and HCV infection. Viral hepatitis B is a worldwide disease caused by HBV. There are more than 400 million people subjected to long-term infection with HBV, which may develop into hepatocellular carcinoma (HCC) and cirrhosis. However, the incidences of liver cancer and cirrhosis developed from HBV infection vary vastly around the world, and incidences of these serious complications may depend on many factors, including gender (the incidence of HCC is higher in men than in women), geographical area (the prevalence of HCC is higher in East Asia, Southeast Asia and sub-Saharan Africa than in other affected areas), environment (such as presence of aflatoxin in food), age of people infected, ethnicity, alcoholism and smoking, etc. (McMahon BJ. The influence of hepatitis B virus genotype and subgenotype on the natural history of chronic hepatitis B. Hepatol Int. 2009;3(2):334-342.). In the process of chronic HBV infection, the inefficient response of immune system to HBV induces low-grade hepatocyte destruction and long-term cytothesis, leading to fibrosis, cirrhosis and steatosis, and finally resulting in HCC (Chisari FV, Isogawa M, Wieland SF. Pathogenesis of hepatitis B virus infection. Pathol Biol (Paris). 2010;58(4):258-266.). The cirrhosis caused by HBV or HCV is mostly treated with antiviral drugs. The nucleoside and nucleotide drugs are recognized as effective drugs against chronic hepatitis B in clinical. These drugs are phosphorylated into triguaiacyl phosphate in vivo on the reverse transcriptase of HBV DNA polymerase to stop the elongation of HBV DNA strand and interfere with the DNA synthesis as the analogue of dNTP, the natural substrate of reverse transcriptase, thereby providing antiviral protection. That is, these drugs are competitive inhibitors of reverse transcriptase. Currently, the nucleoside and nucleotide drugs used can be divided into L-type nucleosides, D-type cyclopentanes and acyclic phosphates, mainly including Entecavir (ETV), Tenofovir Disoproxil Fumarate (TDF), Tenofovir Alafenamide Fumarate (TAF) and Telbivudine (LdT) (Delaney WE 4th, Locamini S, Shaw T. Resistance of hepatitis B virus to antiviral drugs: current aspects and directions for future investigation. Antivir Chem Chemother. 2001;12(1):1-35. ; Caliò R, Villani N, Balestra E, et al. Enhancement of natural killer activity and interferon induction by different acyclic nucleoside phosphonates. Antiviral Res. 1994;23(1):77-89.). It is known that HBV can only infect humans, chimpanzees and tree shrews among all species.

Both hepatitis B and C are infectious hepatitis, which allow spreading through blood, sexual activity and mother-to-child vertical transmission. But, unfortunately, there is no effective vaccine against HCV developed in the medical community. As HCV is an RNA virus mutating at a high rate, it is very difficult to develop the corresponding vaccine in time. in addition, HCV is not capable of infecting animals but humans and chimpanzees, so it is difficult to find suitable animal model for vaccine development. The chronic HCV infection is asymptomatic at the early stage and has little effect in short run, and it may result in cirrhosis, hepatic decompensation, hepatocellular carcinoma and even death. The course of fibrosis may be accelerated by factors such as aging, duration of HCV infection, gender, alcohol intake, obesity, insulin resistance, type 2 diabetes, co-infection with HIV or HBV and host genetics, leading to cirrhosis and even HCC. There are about 180 million people in the world infected with HCV and at risk of developing serious hepatic complications, such as cirrhosis, hepatocellular carcinoma and decompensation. Although some data show that hepatitis C does not increase the overall mortality, it has been assumed that HCV infection has the potential to reduce life expectancy by 8 to 12 years. (Jacobson IM, Davis GL, El-Serag H, Negro F, Trépo C. Prevalence and challenges of liver diseases in patients with chronic hepatitis C virus infection. Clin Gastroenterol Hepatol. 2010;8(1 1): 924-e 117.; Mahale P, Torres HA, Kramer JR, et al. Hepatitis C virus infection and the risk of cancer among elderly US adults: A registry-based case-control study. Cancer. 2017;123(7):1202-1211.)_{∘} The pathogenesis of hepatitis C is still unclear. The HCV replication in hepatocytes causes the hepatocyte structural and functional changes or interferes with the protein synthesis in hepatocyte, which may result in steatosis and necrosis of hepatocytes, indicating that HCV directly damages the liver and plays a role in the pathogenesis. However, the majority of scholars believe that the cellular immunopathological response may play an important role. It is found that, the same as hepatitis B, hepatitis C is also mainly associated with CD3+ T-cell infiltration, and cytotoxic T cells (TC) specifically attack the target hepatocytes infected by HCV and may destroy them. At present, it is known that in the period between liver injury caused by initial HCV infection and establishment of cirrhosis, a plurality of fibrosis pathways are activated due to the continuous interaction between pathogen-related factors. T helper lymphocyte subtype 17(Th17)-secreted cytokines, particularly interleukin-17(IL-17), participate in the activation of fibrosis pathway and the process of cirrhosis. (Paquissi FC. Immunity and Fibrogenesis: The Role of Th17/IL-17 Axis in HBV and HCV-induced Chronic Hepatitis and Progression to Cirrhosis. Front Immunol. 2017;8:1195)_{∘}

In addition to pathogenesis, NASH-related hepatic fibrosis is different from viral hepatitis-related hepatic fibrosis in cellular expression. For example, adiponectin (APN) is a key adipocytokine produced by adipocytes, which plays an important role in diseases associated with modulation of fat function and metabolism. The circulatory APN level significantly decreases in patients with fibrosis induced by chronic NASH and increases in patients with viral hepatitis, and further increases with the fibrosis (The Relation Between Adiponectin and Liver Fibrosis of Nonalcoholic Fatty Liver and Viral Hepatitis, Zhai Wei, Liu Bei; School of Medical Technology, Xi'an Medical College).

According to pathogenesis and cellular expression, different drugs need to be selected for the patients with hepatic fibrosis in clinic, including corticosteroids, colchicine, silymarin for alcoholic hepatitis-associated hepatic fibrosis, interferon for virus-associated hepatic fibrosis, ursodeoxycholic acid-choline, losartan and the like for NASH-associated hepatic fibrosis (Drugs for liver fibrosis, You Hong, Chinese Journal of Hepatology, Volume 16, Issue 3, March 2008). In addition, for treatment of NASH-associated hepatic fibrosis, drugs that regulate blood glucose and lipid are generally used in combination with diet and body weight control, but such drugs are inefficient for hepatic fibrosis associated with viral hepatitis, to be specific, the hepatic fibrosis is not significantly improved.

In contrast to hepatic cirrhosis, hepatic fibrosis is a reversible process. However, hepatic fibrosis, if not controlled and treated, will develop into cirrhosis and finally result in impaired liver function and hepatonecrosis. Once cirrhosis develops, the patients will be at a very high risk of hepatocellular carcinoma (Ali AH, Lindor KD. Obeticholic acid for the treatment of primary biliary cholangitis. Expert Opin Pharmacother. 2016;17(13):1809-1815.), which causes considerable pain and even death, then liver transplantation is necessary for treatment.

However, there is no highly effective drug approved for the patients with hepatic fibrosis associated with viral hepatitis. Therefore, hepatic fibrosis seriously threatens the health of people at home and abroad, and it is really necessary to provide a drug for hepatic fibrosis related to viral hepatitis.

### Summary of the Invention

In order to solve the problem, the present invention provides GLP-1R/GCGR dual target agonist polypeptide derivatives for hepatic fibrosis associated with viral hepatitis.

The inventor has modified the oxyntomudulin (OXM) molecule in the Chinese patent with application number of 201510237027.7 to obtain GLP-1R/GCGR dual target agonists as the oxyntomodulin analogues, which have longer half-life and insulinotropic activity without adverse events, and are capable of being used for treatment of diseases such as diabetes. Further experiments are carried out for the present invention to provide new potency of the GLP-1R/GCGR dual target agonist polypeptide derivatives and their therapeutic uses for indications.

Another object of the present invention is to provide the biological activity and therapeutic uses of the GLP-1R/GCGR dual target agonist polypeptide derivatives in inhibition and improvement of viral hepatitis-associated hepatic fibrosis.

The inventor has demonstrated that the GLP-1R/GCGR dual target agonist polypeptide derivatives significantly improve the hepatic fibrosis caused by HBV infection in humanized mouse model. It is proved that the GLP-1R/GCGR dual target agonist polypeptide derivatives have significant therapeutic effects on hepatic fibrosis and the like caused by viral hepatitis.

Another object of the present invention is to provide new therapeutic uses for indications of the GLP-1R/GCGR dual target agonist polypeptide derivatives. The GLP-1R/GCGR dual target agonist polypeptide derivatives are expected to be used for preventing or treating hepatic fibrosis and the like caused by viral hepatitis.

The present invention relates to GLP-1R/GCGR dual target agonist polypeptide derivatives including the parent peptide represented by the following amino acid sequence: 
His-Xaa2-Gln-Gly-Thr-Phe-Thr-Ser-Asp-Xaa10-Ser-Lys-Xaa13-Leu-Asp-Xa
a16-Xaa17-Xaa18-Ala-Xaa20-Xaa21-Phe-Xaa23-Xaa24-Trp-Leu-Xaa27-Xaa28-X
aa29-Gly-Gly-Pro-Ser-Ser-Gly-Ala-Pro-Pro-Pro- Ser -COR1
 wherein, R₁ = -NH₂;
Xaa2 = Aib or D-Ser;
Xaa10 = Lys or Tyr;
Xaa13 = Lys or Tyr;
Xaa16 = Glu or Lys;
Xaa17 = Lys or Arg;
Xaa18 = Arg or Ala;
Xaa20 = His, Gln or Lys;
Xaa21 = Asp or Glu;
Xaa23 = IIe or Val;
Xaa24 = Glu or Gin;
Xaa27 = Met, Leu or Nle;
Xaa28 = Asn, Asp or Arg;
Xaa29 = Gly or Thr.

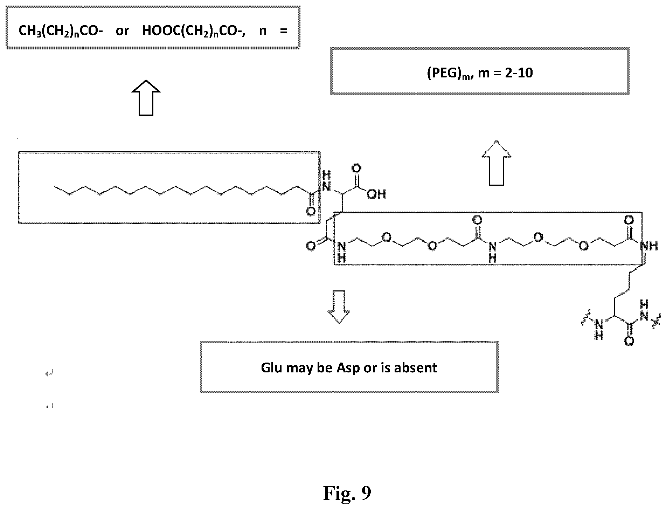

At least one of Xaa10, Xaa16, Xaa17 or Xaa20 is Lys, the side chain of the at least one Lys or the Lys at position 12 of the amino acid sequence is attached to a lipophilic substituent in such a way that a carboxyl group of the lipophilic substituent forms an amide bond with an amino group of a bridging group, the bridging group is attached to the parent peptide by means of a carboxyl group of the bridging group which forms an amide bond with a N-terminal residue of the Lys of the parent peptide, wherein the bridging group is Glu, Asp and/or (PEG)ₘ, and m is an integer of 2 to 10; and the lipophilic substituent is an acyl group selected from a group consisting of CH₃(CH₂)ₙCO- and HOOC(CH₂)ₙCO-, wherein n is an integer of 10 to 24. Referring to Fig. 9, there is shown the attaching mode.

When the position 10, 12, 16, 17, or 20 of the amino acid sequence is Lys, the side chain of the Lys is attached to the lipophilic substituent and the bridging group in one of the following structures:
Lys(PEG₂-PEG₂-CO(CH₂)₁₄CH₃): 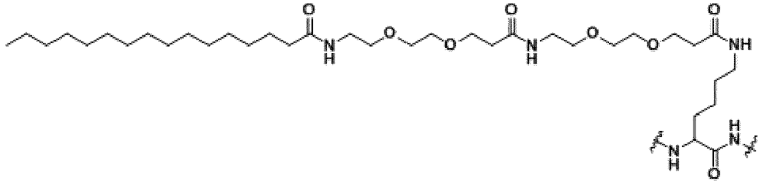
Lys(PEG₂-PEG₂-γGlu-CO(CH₂)₁₄CH₃): 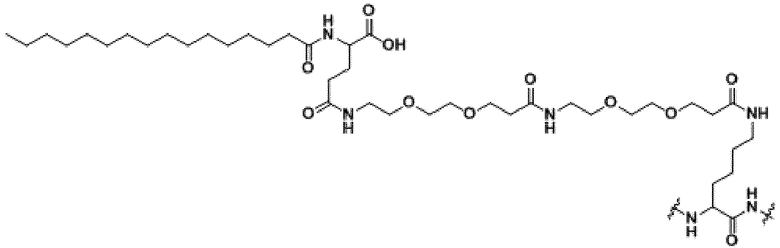
Lys(PEG₂PEG₂-CO(CH₂)₁₄CO₂H): 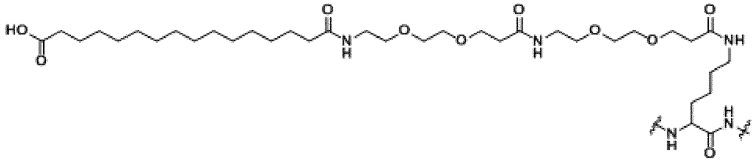
Lys(PEG₂-PEG₂-γGlu-CO(CH₂)₁₄CO₂H): 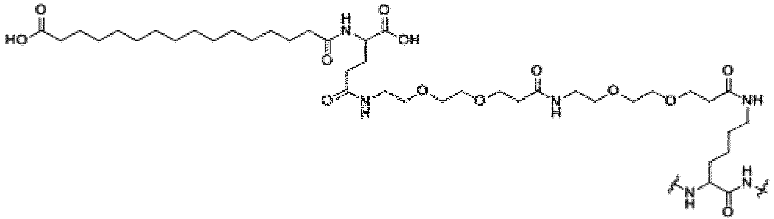
Lys(PEG₂PEG₂-CO(CH₂)₁₆CO₂H): 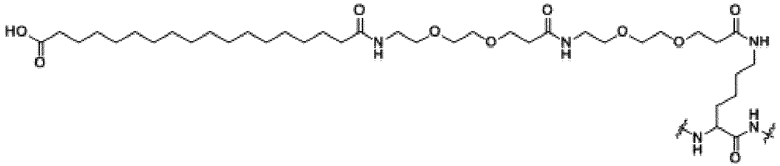
Lys(PEG₂-PEG₂γGlu-CO(CH₂)₁₆CO₂H); 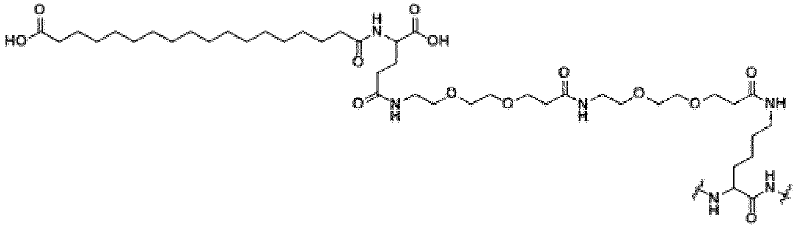
Lys(PEG₂PEG₂CO(CH₂)₁₆CH₃): 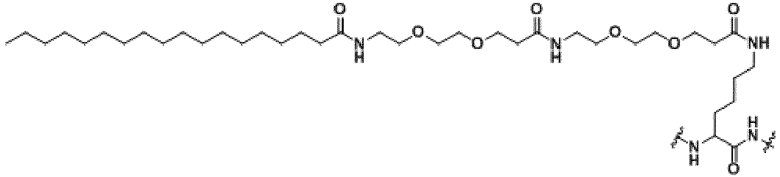
Lys(PEG₂PEG₂-γGlu-CO(CH₂)₁₆CH₃):

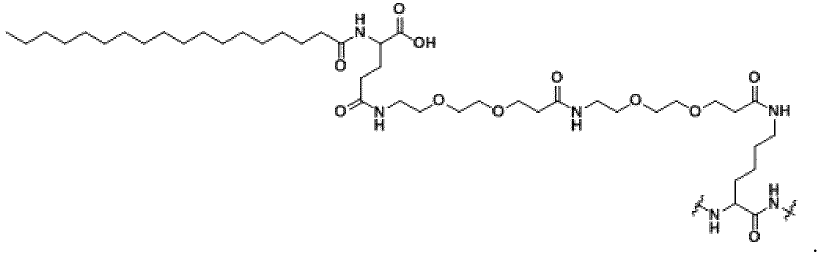

The compounds involved in the present invention are based on the theory that the intramolecular bridges can stabilize the helical structure of the molecule, thus increasing potency and/or selectivity at the GLP-1R or GCGR receptors. The compounds of the present invention may carry one or more intramolecular bridges within the sequence. Each such bridge is formed between the side chains of two amino acid residues which are typically separated by three amino acids in the linear sequence. For example, the bridge may be formed between the side chains of amino acid residue pairs 12 and 16, 16 and 20, 17 and 21, or 20 and 24. The two side chains can be linked to one another through ionic interactions, or by covalent bonds. Thus these pairs of residues may comprise oppositely charged side chains in order to form a salt bridge by ionic interactions. For example, one of the residues may be Glu or Asp, while the other residue may be Lys or Arg. The pairings of Lys and GIu as well as Lys and Asp may also be capable of reacting to form a lactam ring respectively.

According to the present invention, the Lys attached to the lipophilic substituent is replaced with HomoLys, Orn, Dap or Dab.

According to the present invention, the amino acid sequence of the parent peptide is selected from SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24 and SEQ ID NO:25.

The present invention is also to provide a pharmaceutical composition containing the GLP-1R/GCGR dual target agonist polypeptide derivatives of the present invention. The pharmaceutical composition is prepared using the GLP-1R/GCGR dual target agonist polypeptide derivatives as an active ingredient added with pharmaceutically acceptable carriers and/or excipients.

The polypeptide derivatives of the present invention are effective in improvement and treatment of hepatic fibrosis associated with viral hepatitis. The polypeptide derivatives of the present invention may be used for direct or indirect therapy of hepatic fibrosis caused by viral hepatitis and any condition caused or characterized by pathological changes of related hepatic fibrosis.

The person skilled in the art can appreciate that the pharmaceutical composition of the present invention is suitable for various administration routes, such as oral administration, percutaneous administration, intravenous administration, intramuscular administration, topical administration and intranasal administration. According to the used administration route, the pharmaceutical composition containing the polypeptide derivative of the present invention can be formulated into various suitable dosage forms, which comprises an effective amount of at least one polypeptide derivative of the present invention and at least one pharmaceutically acceptable pharmaceutical carrier.

Examples of the suitable dosage forms are tablet, capsule, sugar-coated tablet, granule, oral liquid, syrup, ointment and paste for the skin surface, aerosol, nasal spray and sterile solutions for injection.

The pharmaceutical composition containing the polypeptide derivative of the present invention may be prepared into solution or lyophilized powder for parenteral administration. Before use, an appropriate solvent or other pharmaceutically acceptable carrier may be added to reprepare the powder, and liquid formula is generally buffer, osmotic solution and aqueous solution.

The dosage of the polypeptide derivative of the present invention in the pharmaceutical composition may vary in a wide range, which can be easily determined by those skilled in the art according to certain factors such as type of the disease, the severity of disease, the body weight, the dosage form and the administration route.

The advantages of the present invention are as follows:

As alcohol abuse, NASH and viral hepatitis play completely different roles in pathogenesis of hepatic fibrosis, the fibrosis should be treated according to different principles. In the prior art, there is no drug or compound for fibrosis caused by two or all of the above 3 factors. The GLP-1R/GCGR dual target agonist polypeptide derivatives of the present invention also provide a use for hepatic fibrosis associated with viral hepatitis. The GLP-1R/GCGR dual target agonist polypeptide derivatives of the present invention have significant improvement and efficacy on hepatic fibrosis of virus-infected humanized mice. In addition, the GLP-1R/GCGR dual target agonist polypeptide derivatives of the present invention have good potency; show good stability in pharmacokinetics experiment of drugs, which are convenient for production on large scale at low cost; and have lower toxicity, longer safe window and smaller amount compared with small-molecule compound.

In particular embodiments, the following GLP-1R/GCGR dual target agonist polypeptide derivatives are related, having the following sequences:
**Compound 1 (SEQ ID NO:1):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)14CH3)-Arg-Arg-Ala-Gln-Asp-Phe-Val-Gln-Trp-Leu-Met-Asn-
Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)14CH3)-RRAQDFVQ
WLMNTGGPSSGAPPPS-NH2
**Compound 2 (SEQ ID NO:2):** 
His-(D-Ser)-Gln-Gly-Thr-Phe-Thr-Ser-Asp-Tyr-Ser-Lys-Tyr-Leu-Asp-Glu-Lys(PE
G2-PEG2-γGlu-CO(CH2)16CO2H)-Arg-Ala-Gln-Asp-Phe-Val-Gln-Trp-Leu-Met-As
n-Thr-Gly-Gly-Pro-Ser-Ser-Gly-Ala-Pro-Pro-Pro-Ser-NH2
H-(d-S)-QGTFTSDYSKYLDE-K(PEG2-PEG2-γGlu-CO(CH2)16CO2H)-RAQDFV
QWLMNTGGPSSGAPPPS-NH2
**Compound 3 (SEQ ID NO:3):** 
His-Aib-Gln-Gly-Thr-Phe-Thr-Ser-Asp-Lys(PEG2-PEG2-γGlu-CO(CH2)14CO2H)-S
er-Lys-Tyr-Leu-Asp-Glu-Arg-Arg-Ala-Gln-Asp-Phe-Val-Gln-Trp-Leu-Leu-Asp-G
ly-Gly- Gly-Pro-Ser-Ser-Gly-Ala-Pro-Pro-Pro-Ser-NH2
H-Aib-QGTFTSD-K(PEG2-PEG2-γGlu-CO(CH2)14CO2H)-SKYLDERRAQDFVQ
WLLDGGGPSSGAPPPS-NH2
**Compound 4 (SEQ ID NO:4):** 
His-Aib-Gln-Gly-Thr-Phe-Thr-Ser-Asp-Lys(PEG₂-PEG₂-γGlu-CO(CH₂)₁₆CH₃)-Ser
-Lys-Tyr-Leu-Asp-Glu-Arg-Arg-Ala-Gln-Asp-Phe-Val-Gln-Trp-Leu-Leu-Asp-Gly
-Gly-Pro-Ser-Ser-Gly-Ala-Pro-Pro-Pro-Ser-NH₂
H-Aib-QGTFTSD-K(PEG₂-PEG₂-γGlu-CO(CH₂)₁₆CH₃)-SKYLDERRAQDFVQW
LLDGGPSSGAPPPS-NH₂
Compound 5 (SEQ ID NO:5): 
His-Aib-Gln-Gly-Thr-Phe-Thr-Ser-Asp-Lys(PEG2-PEG2-γGlu-CO(CH2)16CO2H)-S
er-Lys-Tyr-Leu-Asp-Glu-Arg-Arg-Ala-Gln-Asp-Phe-Val-Gln-Trp-Leu-Leu-Asp-G
ly-Gly- Gly-Pro-Ser-Ser-Gly-Ala-Pro-Pro-Pro-Ser-NH2
H-Aib-QGTFTSD-K(PEG2-PEG2-γGlu-CO(CH2)16CO2H)-SKYLDERRAQDFVQ
WLLDGGGPSSGAPPPS-NH2
**Compound 6 (SEQ ID NO:6):** 
His-Aib-Gln-Gly-Thr-Phe-Thr-Ser-Asp-Lys(PEG2-PEG2-γGlu-CO(CH2)14CH3)-Ser
-Lys-Tyr-Leu-Asp-Glu-Arg-Arg-Ala-Gln-Asp-Phe-Val-Gln-Trp-Leu-Leu-Asp-Gly
-Gly- Gly-Pro-Ser-Ser-Gly-Ala-Pro-Pro-Pro-Ser-NH2
H-Aib-QGTFTSD-K(PEG2-PEG2-γGlu-CO(CH2)14CH3)-SKYLDERRAQDFVQW
LLDGGGPSSGAPPPS-NH2
**Compound 7 (SEQ ID NO:7):** 
His-(D-Ser)-Gln-Gly-Thr-Phe-Thr-Ser-Asp-Tyr-Ser-Lys-Tyr-Leu-Asp-Lys-Lys(PE
G₂-PEG₂-γGlu-CO(CH₂)₁₆CO₂H)-Ala-Ala-His-Asp-Phe-Val-Glu-Trp-Leu-Leu-Arg
-Thr-Gly-Gly-Pro-Ser-Ser-Gly-Ala-Pro-Pro-Pro-Ser-NH₂
H-(*d*-S)-QGTFTSDYSKYLDK-K(PEG₂-PEG₂-γGlu-CO(CH₂)₁₆CO₂H)-AAHDFV
EWLLRTGGPSSGAPPPS-NH₂
**Compound 8 (SEQ ID NO:8):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)16CO2H)-Glu-Phe-Ile-Glu-Trp-Leu-Leu-Arg-
Thr-Gly-Gly-Pro-Ser-Ser-Gly-Ala-Pro-Pro-Pro-Ser-NH2
H-(d-S)-QGTFTSDYSKYLDEKAA-K(PEG2-PEG2-γGlu-CO(CH2)16CO2H)-EFIE
WLLRTGGPSSGAPPPS-NH2
**Compound 9 (SEQ ID NO:9):** His-(D-Ser)-Gln-Gly-Thr-Phe-Thr-Ser-Asp-Tyr-Ser-Lys-Tyr-Leu-Asp-Glu-Lys(PE 
G2-PEG2-γGlu-CO(CH2)16CO2H)-Ala-Ala-His-Asp-Phe-Val-Glu-Trp-Leu-Leu-Asn
-Gly-Gly- Gly-Pro-Ser-Ser-Gly-Ala-Pro-Pro-Pro-Ser-NH2
H-(d-S)-QGTFTSDYSKYLDE-K(PEG2-PEG2-γGlu-CO(CH2)16CO2H)-AAHDFV
EWLLNGGGPSSGAPPPS-NH2
**Compound 10 (SEQ ID NO:10):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)14CH3)-Glu-Phe-Ile-Glu-Trp-Leu-Leu-Arg-
-Gly-Gly-Gly-Pro-Ser-Ser-Gly-Ala-Pro-Pro-Pro-Ser-NH2
H-(d-S)-QGTFTSDYSKYLDEKAA-K(PEG2-PEG2-γGlu-CO(CH2)14CH3)-EFIEW
LLRGGGPSSGAPPPS-NH2
**Compound 11 (SEQ ID NO:11):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)16CH3)-Glu-Phe-Ile-Glu-Trp-Leu-Leu-Arg-
-Gly-Gly-Gly-Pro-Ser-Ser-Gly-Ala-Pro-Pro-Pro-Ser-NH2
H-(d-S)-QGTFTSDYSKYLDEKAA-K(PEG2-PEG2-γGlu-CO(CH2)16CH3)-EFIEW
LLRGGGPSSGAPPPS-NH2
**Compound 12 (SEQ ID NO:12):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)16CO2H)-Glu-Phe-Ile-Glu-Trp-Leu-Leu-Asn-
Gly-Gly- Gly-Pro-Ser-Ser-Gly-Ala-Pro-Pro-Pro-Ser-NH2
H-(d-S)-QGTFTSDYSKYLDEKAA-K(PEG2-PEG2-γGlu-CO(CH2)16CO2H)-EFIE
WLLNGGGPSSGAPPPS-NH2
**Compound 13 (SEQ ID NO:13):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)14CO2H)-Glu-Phe-Ile-Glu-Trp-Leu-Leu-Asn-
Gly-Gly- Gly-Pro-Ser-Ser-Gly-Ala-Pro-Pro-Pro-Ser-NH2
 
H-(d-S)-QGTFTSDYSKYLDEKAA-K(PEG2-PEG2-γGlu-CO(CH2)14CO2H)-EFIE
WLLNGGGPSSGAPPPS-NH2
**Compound 14 (SEQ ID NO:14):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG2-PEG2-γGlu-CO(CH2)16CH3)-Glu-Phe-Ile-Glu-Trp-Leu-Leu-Asn-G
ly-Gly- Gly-Pro-Ser-Ser-Gly-Ala-Pro-Pro-Pro-Ser-NH2
H-(d-S)-QGTFTSDYSKYLDEKAA-K(PEG2-PEG2-γGlu-CO(CH2)16CH3)-EFIEW
LLNGGGPSSGAPPPS-NH2
**Compound 15 (SEQ ID NO:15):** 
His-(D-Ser)-Gln-Gly-Thr-Phe-Thr-Ser-Asp-Tyr-Ser-Lys-Tyr-Leu-Asp-Glu-Lys-Al
a-Ala-Lys(PEG₂-PEG₂-γGlu-CO(CH₂)₁₄CH₃)-Glu-Phe-Ile-Glu-Trp-Leu-Leu-Asn-G
ly-Gly- Gly-Pro-Ser-Ser-Gly-Ala-Pro-Pro-Pro-Ser-NH₂
H-(*d*-S)-QGTFTSDYSKYLDEKAA-K(PEG₂-PEG₂-γGlu-CO(CH₂)₁₄CH₃)-EFIEW
LLNGGGPSSGAPPPS-NH₂
**Compound 16 (SEQ ID NO:16):** 
His-(D-Ser)-Gln-Gly-Thr-Phe-Thr-Ser-Asp-Tyr-Ser-Lys-Tyr-Leu-Asp-Lys-Lys(PE
G2-PEG2-γGlu-CO(CH2)14CH3)-Arg-Ala-Gln-Asp-Phe-Val-Gln-Trp-Leu-Met-Asn-
Thr-Gly-Gly-Pro-Ser-Ser-Gly-Ala-Pro-Pro-Pro-Ser-NH2
H-(d-S)-QGTFTSDYSKYLDK-K(PEG2-PEG2-γGlu-CO(CH2)14CH3)-RAQDFVQ
WLMNTGGPSSGAPPPS-NH2
**Compound 17 (SEQ ID NO:17):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)16CO2H)-Arg-Arg-Ala-Gln-Asp-Phe-Val-Gln-Trp-Leu-Met-As
n-Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)16CO2H)-RRAQDFV
QWLMNTGGPSSGAPPPS-NH2
**Compound 18 (SEQ ID NO:18):** 
His-(D-Ser)-Gln-Gly-Thr-Phe-Thr-Ser-Asp-Tyr-Ser-Lys-Tyr-Leu-Asp-Lys(PEG2-P
EG2-CO(CH2)16CO2H)-Arg-Arg-Ala-Gln-Asp-Phe-Val-Gln-Trp-Leu-Met-Asn-Thr-
Gly-Gly-Pro-Ser-Ser-Gly-Ala-Pro-Pro-Pro-Ser-NH2
H-(d-S)-QGTFTSDYSKYLD-K(PEG2-PEG2-CO(CH2)16CO2H)-RRAQDFVQWL
MNTGGPSSGAPPPS-NH2
**Compound 19 (SEQ ID NO:19):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)16CH3)-Arg-Arg-Ala-Gln-Asp-Phe-Val-Gln-Trp-Leu-Met-Asn-
Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)16CH3)-RRAQDFVQ
WLMNTGGPSSGAPPPS-NH2
**Compound 20 (SEQ ID NO:20):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)14CH3)-Arg-Arg-Ala-Gln-Asp-Phe-Val-Gln-Trp-Leu-Nle
-Asn-Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)14CH3)-RRAQDFVQ
WL-Nle-NTGGPSSGAPPPS-NH2
**Compound 21 (SEQ ID NO:21):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)16CO2H)-Arg-Arg-Ala-Gln-Asp-Phe-Val-Gln-Trp-Leu-Nle-Asn
-Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)16CO2H)-RRAQDFV
QWL-Nle-NTGGPSSGAPPPS-NH2
**Compound 22 (SEQ ID NO:22):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)16CO2H)-Arg-Arg-Ala-Gln-Asp-Phe-Val-Gln-Trp-Leu-Leu-As
n-Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)16CO2H)-RRAQDFV
QWLLNTGGPSSGAPPPS-NH2
**Compound 23 (SEQ ID NO:23):** 
His-(D-Ser)-Gln-Gly-Thr-Phe-Thr-Ser-Asp-Tyr-Ser-Lys-Tyr-Leu-Asp-Lys(PEG2-P
EG2-γGlu-CO(CH2)14CH3)-Arg-Arg-Ala-Gln-Asp-Phe-Val-Gln-Trp-Leu-Leu-Asn-
Thr-Gly-Gly-Pro-Ser-Ser-Gly-Ala-Pro-Pro-Pro-Ser-NH2
H-(d-S)-QGTFTSDYSKYLD-K(PEG2-PEG2-γGlu-CO(CH2)14CH3)-RRAQDFVQ
WLLNTGGPSSGAPPPS-NH2
**Compound 24 (SEQ ID NO:24):** 
His-(D-Ser)-Gln-Gly-Thr-Phe-Thr-Ser-Asp-Tyr-Ser-Lys-Tyr-Leu-Asp-Lys(PEG2-P
EG2-CO(CH2)16CO2H)-Arg-Arg-Ala-Gln-Asp-Phe-Val-Gln-Trp-Leu-Nle-Asn-Thr-
Gly-Gly-Pro-Ser-Ser-Gly-Ala-Pro-Pro-Pro-Ser-NH2
H-(d-S)-QGTFTSDYSKYLD-K(PEG2-PEG2-CO(CH2)16CO2H)-RRAQDFVQWL-
Nle-NTGGPSSGAPPPS-NH2
**Compound 25 (SEQ ID NO:25):**

His-(D-Ser)-Gln-Gly-Thr-Phe-Thr-Ser-Asp-Tyr-Ser-Lys-Tyr-Leu-Asp-Lys(PEG2-P
EG2-CO(CH2)14CH3)-Arg-Arg-Ala-Gln-Asp-Phe-Val-Gln-Trp-Leu-Leu-Asn-Thr-G
ly-Gly-Pro-Ser-Ser-Gly-Ala-Pro-Pro-Pro-Ser-NH2
H-(d-S)-QGTFTSDYSKYLD-K(PEG2-PEG2-CO(CH2)14CH3)-RRAQDFVQWLL
NTGGPSSGAPPPS-NH2

**The abbreviations used in the present invention are defined as follows:**
Boc is tert-butyloxycarbonyl, Fmoc is fluorenylmethoxycarbonyl, t-Bu is tert-butyl, ivDDe is 1-(4,4-dimethyl-2,6-dioxo-cyclohexylidene)-3-methyl-butyl removal and lipophilic substituent, resin is resin, TFA is trifluoroacetic acid, EDT is 1,2-ethanedithiol, Phenol is phenol, FBS is fetal bovine serum, BSA is bovine serum albumin, HPLC is high performance liquid chromatography, GLP-1R is glucagon-like peptide-1 receptor, GCGR is glucagon receptor, GLP-1 is glucagon-like peptide, mPEG is mono-methoxy-polyethylene diol, OXM is oxyntomodulin, His is histidine, Ser is serine, D-Ser is D-serine, Gln is glutamine, Gly is glycine, Glu is glutamic acid, Ala is alanine acid, Thr is threonine, Lys is lysine, Arg is arginine, Tyr is tyrosine, Asp is aspartic acid, Trp is tryptophan, Phe is phenylalanine, IIe is isoleucine, Leu is leucine, Cys is cysteine, Pro is proline, Val is valine, Met is methionine, Asn is asparagines, HomoLys is homolysine, Orn is ornithine, Dap is diaminopimelic acid, Dab is 2,4-diaminobutyric acid, NIe is norleucine, Aib is 2-aminoisobutyric acid and AEEA is [2-[2-(amido)ethoxy]ethoxy]acetic acid.

### Description of the Drawings

Embodiments of the present invention are described in detail below with reference to the attached drawings, wherein:
**Fig. 1** is a FG/SR staining pathological section of HBV-infected humanized mouse liver in Example 3.
**Fig. 2** is a statistical histogram of FG/SR staining pathological section of HBV-infected humanized mouse liver in Example 3 (****: compared with a control group (99% CI, p<0.0001)).
**Fig. 3** is an H&E staining pathological section of HBV-infected humanized mouse liver in Example 3.
**Fig. 4** is a statistical histogram of H&E staining pathological section of HBV-infected humanized mouse liver in Example 3 (^{∗∗∗∗}: compared with the control group (99% CI, p<0.0001)).
**Fig. 5** is an α-SMA immunohistochemical fluorescence (IF) diagram of HBV-infected humanized mouse liver in Example 3.
**Fig. 6** is an α-SMA IF statistical histogram of HBV-infected humanized mouse liver in Example 3 (^{∗∗∗∗}: compared with the control group (99% CI, p<0.0001)).
**Fig. 7** is a statistical histogram of serum ALT determination in HBV-infected humanized mouse in Example 3 (^{∗∗∗∗}: compared with the control group (99% CI, p<0.0001)).
**Fig. 8** is a statistical histogram of human IFN-y determination in serum of HBV-infected humanized mouse in Example 3 (^{∗∗∗∗}: compared with the control group (99% CI, p<0.0001)).
**Fig. 9** is a schematic diagram of the connection between the bridging group and the peptide chain in the compound of the present invention.
**Fig. 10** is a gating method and humanized immune map of fluorescence activated cell sorting (FACs) in humanized mouse in Example 3.

### Detailed Description of the Invention

The embodiments of the present invention will be described in detail hereafter in conjunction with the examples, but those skilled in the art will appreciate that the following examples are only presented for purposes of illustration of the present invention and shall not be construed as restriction to the scope. Unless otherwise specified in the embodiments, the examples were carried out according to conventional conditions or the conditions recommended by manufacturers. Unless otherwise specified, the reagents or instruments used are commercially available

### Example 1: Synthesis of polypeptide compound

### Materials:

All amino acids were purchased from NovaBiochem. Unless otherwise specified, all other reagents were analytical reagent, purchased from Sigma Company. Protein Technologies PRELUDE 6-channel polypeptide synthesizer was used. Phenomenex Luna C18 preparative column (46 mm x 250 mm) was used for purification of the polypeptides. High performance liquid chromatography instrument was manufactured by Waters Company. MS analysis was determined using Agilent mass spectrometer.

### Synthetic method of polypeptide compounds of the present invention is illustrated by taking the polypeptide compound 1 as an example:

### Structure sequence of compound 1:

His-(D-Ser)-Gln-Gly-Thr-Phe-Thr-Ser-Asp-Tyr-Ser-Lys-Tyr-Leu-Asp-Lys(PEG₂-P
EG₂-γGlu-CO(CH₂)₁₄CH₃)-Arg-Arg-Ala-Gln-Asp-Phe-Val-Gln-Trp-Leu-Met-Asn-
Thr-Gly-Gly-Pro-Ser-Ser-Gly-Ala-Pro-Pro-Pro-Ser-NH₂

### a) Main peptide chain assembly:

The following polypeptide in a scale of 0.25 mmol was synthesized on a CS336X polypeptide synthesizer (CS Bio American Company) according to Fmoc/*t*-Bu strategy:
Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(ivDde)-Arg(Pbf)-Arg(Pbf)-Ala-Gln(Trt)-Asp(OtBu)-Phe-Val-Gln(Trt)-Trp(Boc)-Leu-Met-Asn(Trt)-Thr(t-Bu)-Gly-Gly-Pro-Ser(t-Bu)-Ser(t-Bu)-Gly-Ala-Pro-Pro-Pro-Ser(t-B u)-rink amide resin.
   (1) Step 1: 0.75 g of Rink amide MBHA-LL resin (Novabiochem, loading 0.34 mmol/g) was swelled in dichloromethane (DCM) for 1 hour, and the resin was fully washed with *N,N*-dimethylformamide (DMF) three times.
   (2) Step 2: The procedure reaction was performed using Rink amide resin as carrier, the mixture of 6-chlorobenzotriazole-1,1,3,3-tetramethyluronium hexafluorophosphate (HCTU), organic base *N,N*-diisopropylethylamine (DIEPA) at a molar ratio of 1:1 as coupling agent, and *N,N*-dimethylformamide (DMF) as solvent, the condensation reactions were performed to successively bind
Fmoc-Ser(t-Bu)-OH, Fmoc-Pro-OH (3x), Fmoc-Ala-OH, Fmoc-Gly-OH, Fmoc-Ser(t-Bu)-OH (2x), Fmoc-Pro-OH, Fmoc-Gly-OH (2x), Fmoc-Thr(t-Bu)-OH, Fmoc-Asn(Trt)-OH, Fmoc-Met-OH, Fmoc-Leu-OH, Fmoc-Trp(Boc)-OH, Fmoc-Glu(OtBu)-OH, Fmoc-Val-OH, Fmoc-Phe-OH, Fmoc-Asp(OtBu)-OH, Fmoc-Gln(Trt)-OH, Fmoc-Ala-OH, Fmoc-Arg(Pbf)-OH (2x), Fmoc-Lys(ivDde)-OH, Fmoc-Asp(OtBu)-OH, Fmoc-Leu-OH, Fmoc-Tyr(t-Bu)-OH, Fmoc-Lys(Boc)-OH, Fmoc-Ser(t-Bu)-OH, Fmoc-Tyr(t-Bu)-OH, Fmoc-Asp(OtBu)-OH, Fmoc-Ser(t-Bu)-OH, Fmoc-Thr(t-Bu)-OH, Fmoc-Phe-OH, Thr(t-Bu)-OH, Fmoc-Gly-OH, Fmoc-Gln(Trt)-OH, Fmoc-D-Ser(t-Bu)-OH, Boc-His(Boc)-OH, obtaining:
   Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(ivDde)-Arg(Pbf)-Arg(Pbf)-Ala-Gln(Trt)-Asp(OtBu)-Phe-Val-Gln(Trt)-Trp(Boc)-Leu-Met-Asn(Trt)-Thr(t-Bu)-Gly-Gly-Pro-Ser(t-Bu)-Ser(t-Bu)-Gly-Ala-Pro-Pro-Pro-Ser(t-B u)-rink amide resin. Subsequently, the resin was fully washed with *N,N*-dimethylformamide (DMF), dichloromethane (DCM), methanol, dichloromethane (DCM), *N,N*-dimethylformamide (DMF) three times.

In the reaction, 1) the amount of the first amino acid Fmoc-Ser(t-Bu)-OH and the amount of the resin was at a ratio of 1:1-6:1; and 2) in each of the subsequent condensation reactions, each of the amount of Fmoc-protected amino acid, 6-chlorobenzotriazole-1,1,3,3-tetramethyluronium hexafluorophosphate (HCTU), organic base *N,N*-diisopropylethylamine (DIEPA) was excess by 2-8 times, the reaction time was 1-5 hours.

### b) Removal of 1-(4,4-dimethyl-2,6-dioxo-cyclohexylidene)-3-methyl-butyl (ivDde) and introduction of lipophilic substituent:

The resin was washed twice in the solution of *N,N*-dimethylformamide (DMF) /dichloromethane (DCM) = 1: 1 (volume ratio), and added with freshly prepared 3.0% hydrazine hydrate in *N,N*-dimethylformamide (DMF). The mixture was shaken at room temperature for 10-30 minutes, and then filtered. The hydrazine treatment step was repeated five times to obtain:
Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys-Arg(Pbf) -Arg(Pbf)-Ala-Gln(Trt)-Asp(OtBu)-Phe-Val-Gln(Trt)-Trp(Boc)-Leu-Met-Asn(Trt)-Thr(t-Bu)-Gly-Gly-Pro-Ser(t-Bu)-Ser(t-Bu)-Gly-Ala-Pro-Pro-Pro-Ser(t-Bu)-rink amide resin. Subsequently, the resin was fully washed with *N,N*-dimethylformamide (DMF), dichloromethane (DCM), methanol, dichloromethane (DCM), *N,N-*dimethylformamide (DMF) three times.

Thereto was added an *N,N*-dimethylformamide (DMF) mixed coupling solution of FmocNH-PEG₂-OH (Quanta BioDesign), 2-(7-azo BTA)-N,N,N',N'-tetramethyluronium hexafluorophosphate (HATU) and diisopropylethyl amine (DIEPA) (5 times excess of each), shaken for 2 hours, and filtrated. Subsequently, the resin was fully washed with *N,N*-dimethylformamide (DMF), dichloromethane (DCM), methanol, dichloromethane (DCM), *N,N*-dimethylformamide (DMF) in sequence (three times of each) to obtain:
Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(Fmoc-P EG₂)-Arg(Pbf)-Arg(Pbf)-Ala-Gln(Trt)-Asp(OtBu)-Phe-Val-Gln(Trt)-Trp(Boc)-Leu -Met-Asn(Trt)-Thr(t-Bu)-Gly-Gly-Pro-Ser(t-Bu)-Ser(t-Bu)-Gly-Ala-Pro-Pro-Pro-S er(t-Bu)-rink amide resin. Subsequently, the resin was fully washed with N,N-dimethylformamide (DMF), dichloromethane (DCM), methanol, dichloromethane (DCM), *N,N*-dimethylformamide (DMF) three times.

A Fmoc group was removed with 20% piperidine/N,N-dimethylformamide (DMF) solution (30 minutes, removal twice). Thereto was added the *N,N*-dimethylformamide (DMF) mixed coupling solution of Fmoc-PEG₂-OH, 2-(7-azo BTA)-N,N,N',N'-tetramethyluronium hexafluorophosphate (HATU) and diisopropylethyl amine (DIEPA) (5 times excess of each) to carry out the coupling reaction to obtain:
Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(Fmoc-P EG₂-PEG₂)-Arg(Pbf)-Arg(Pbf)-Ala-Gln(Trt)-Asp(OtBu)-Phe-Val-Gln(Trt)-Trp(Bo c)-Leu-Met-Asn(Trt)-Thr(t-Bu)-Gly-Gly-Pro-Ser(t-Bu)-Ser(t-Bu)-Gly-Ala-Pro-Pro -Pro-Ser(t-Bu)-rink amide resin. Subsequently, the resin was fully washed with *N,N*-dimethylformamide (DMF), dichloromethane (DCM), methanol, dichloromethane (DCM), *N,N*-dimethylformamide (DMF) three times.

The Fmoc group was removed with 20% piperidine/*N,N*-dimethylformamide (DMF) solution (30 minutes, removal twice). Fmoc-yGlu-OtBu was sequentially coupled under conventional conditions and palmitic acid was added to obtain:
Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(PEG₂-P EG₂-C16)-Arg(Pbf)-Arg(Pbf)-Ala-Gln(Trt)-Asp(OtBu)-Phe-Val-Gln(Trt)-Trp(Boc) -Leu-Met-Asn(Trt)-Thr(t-Bu)-Gly-Gly-Pro-Ser(t-Bu)-Ser(t-Bu)-Gly-Ala-Pro-Pro-P ro-Ser(t-Bu)-rink amide resin. The resin was fully washed with *N,N*-dimethylformamide (DMF), dichloromethane (DCM), Methanol and dichloromethane (DCM) three times respectively, and dried under vacuum.

### c) Removal of fully protected polypeptide:

Boc-His(Boc)-D-Ser(t-Bu)-Gln(OtBu)-Gly-Thr(t-Bu)-Phe-Thr(t-Bu)-Ser(tBu) -Asp(OtBu)-Tyr(t-Bu)-Ser(t-Bu)-Lys(Boc)-Tyr(t-Bu)-Leu-Asp(OtBu)-Lys(PEG2-P EG2-C16)-Arg(Pbf)-Arg(Pbf)-Ala-Gln(Trt)-Asp(OtBu)-Phe-Val-Gln(Trt)-Trp(Boc) -Leu-Met-Asn(Trt)-Thr(t-Bu)-Gly-Gly-Pro-Ser(t-Bu)-Ser(t-Bu)-Gly-Ala-Pro-Pro-P ro-Ser(t-Bu)-rink amide resin was added with a cutting fluid TFA/Phenol/thioanisole/EDT/H₂O (at volume ratio of 82.5:5:5:2.5:5) and heated, controlling the temperature of cracking solution at 25 °C, and reacted for 2.5 hours. After filtration, the filter cake was washed three times with a small amount of cracking solution, and the filtrates were collected together. The filtrate was slowly poured into diethyl ether with stirring, allowed to stand for more than 2 hours to precipitate completely. The precipitate was centrifuged and washed with diethyl ether three times to obtain crude compound:
His-(D-Ser)-Gln-Gly-Thr-Phe-Thr-Ser-Asp-Tyr-Ser-Lys-Tyr-Leu-Asp-Lys(P EG₂-PEG₂-γGlu-CO(CH₂)₁₄CH₃)-Arg-Arg-Ala-Gln-Asp-Phe-Val-Gln-Trp-Leu-Me t-Asn-Thr-Gly-Gly-Pro-Ser-Ser-Gly-Ala-Pro-Pro-Pro-Ser-NH₂

### d) Purification of the polypeptide compound:

The obtained crude product was dissolved in a solution of acetonitrile (ACN):H₂O = 1:1 (volume ratio), and purified by preparative HPLC on a 5.0 mm reverse-phase C18-packed 46 mm x 250 mm column. 30% acetonitrile (containing 0.05% trifluoroacetic acid)/H₂O (containing 0.05% trifluoroacetic acid) were taken as starting materials to elute the column at a gradient (the proportion of acetonitrile is added at a rate of 1.33%/min) for 30 minutes at 15 mL/min, the components containing peptide were collected and lyophilized, so as to obtain a pure product with a HPLC purity greater than 95%. The isolated product was analyzed by LC-MS.

Based on the above synthetic steps, the polypeptide compounds synthesized in the present invention include (Table 1):

**Table 1. Structures of polypeptide compounds synthesized in the examples of the present invention**

| **(SEQ ID NO:)** | **Sequence** |
|---|---|
| **1** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)14CH3)-Arg-Arg-Ala-Gln -Asp-Phe-Val-Gln-Trp-Leu-Met-Asn-Thr-Gly-Gly-Pro-Ser-Ser- Gly-Ala-Pro-Pro-Pro-Ser-NH2 |
| **2** | His-(D-Ser)-Gln-Gly-Thr-Phe-Thr-Ser-Asp-Tyr-Ser-Lys-Tyr-Le u-Asp-Glu-Lys(PEG2-PEG2-γGlu-CO(CH2)16CO2H)-Arg-Ala-Gl n-Asp-Phe-Val-Gln-Trp-Leu-Met-Asn-Thr-Gly-Gly-Pro-Ser-Ser -Gly-Ala-Pro-Pro-Pro-Ser-NH2 |
| **3** | His-Aib-Gln-Gly-Thr-Phe-Thr-Ser-Asp-Lys(PEG2-PEG2-γGlu-C O(CH2)14CO2H)-Ser-Lys-Tyr-Leu-Asp-Glu-Arg-Arg-Ala-Gln-A sp-Phe-Val-Gln-Trp-Leu-Leu-Asp-Gly-Gly-Gly-Pro-Ser-Ser-Gl y-Ala-Pro-Pro-Pro-Ser-NH2 |
| **4** | His-Aib-Gln-Gly-Thr-Phe-Thr-Ser-Asp-Lys(PEG₂-PEG₂-γGlu-C O(CH₂)₁₆CH₃)-Ser-Lys-Tyr-Leu-Asp-Glu-Arg-Arg-Ala-Gln-Asp -Phe-Val-Gln-Trp-Leu-Leu-Asp-Gly-Gly-Pro-Ser-Ser-Gly-Ala- Pro-Pro-Pro-Ser-NH₂ |
| **5** | His-Aib-Gln-Gly-Thr-Phe-Thr-Ser-Asp-Lys(PEG2-PEG2-γGlu-C O(CH2)16CO2H)-Ser-Lys-Tyr-Leu-Asp-Glu-Arg-Arg-Ala-Gln-A sp-Phe-Val-Gln-Trp-Leu-Leu-Asp-Gly-Gly-Gly-Pro-Ser-Ser-Gl |
| | y-Ala-Pro-Pro-Pro-Ser-NH2 |
| **6** | His-Aib-Gln-Gly-Thr-Phe-Thr-Ser-Asp-Lys(PEG₂-PEG₂-γGlu-C O(CH₂)₁₄CH₃)-Ser-Lys-Tyr-Leu-Asp-Glu-Arg-Arg-Ala-Gln-Asp -Phe-Val-Gln-Trp-Leu-Leu-Asp-Gly-Gly-Gly-Pro-Ser-Ser-Gly- Ala-Pro-Pro-Pro-Ser-NH₂ |
| **7** | His-(D-Ser)-Gln-Gly-Thr-Phe-Thr-Ser-Asp-Tyr-Ser-Lys-Tyr-Le u-Asp-Lys-Lys(PEG2-PEG2-γGlu-CO(CH2)16CO2H)-Ala-Ala-Hi s-Asp-Phe-Val-Glu-Trp-Leu-Leu-Arg-Thr-Gly-Gly-Pro-Ser-Ser- Gly-Ala-Pro-Pro-Pro-Ser-NH2 |
| **8** | His-(D-Ser)-Gln-Gly-Thr-Phe-Thr-Ser-Asp-Tyr-Ser-Lys-Tyr-Le u-Asp-Glu-Lys-Ala-Ala-Lys(PEG2-PEG2-γGlu-CO(CH2)16CO2H )-Glu-Phe-Ile-Glu-Trp-Leu-Leu-Arg-Thr-Gly-Gly-Pro-Ser-Ser- Gly-Ala-Pro-Pro-Pro-Ser-NH2 |
| **9** | His-(D-Ser)-Gln-Gly-Thr-Phe-Thr-Ser-Asp-Tyr-Ser-Lys-Tyr-Le u-Asp-Glu-Lys(PEG₂-PEG₂-γGlu-CO(CH₂)₁₆CO₂H)-Ala-Ala-Hi s-Asp-Phe-Val-Glu-Trp-Leu-Leu-Asn-Gly-Gly-Gly-Pro-Ser-Ser -Gly-Ala-Pro-Pro-Pro-Ser-NH₂ |
| **10** | His-(D-Ser)-Gln-Gly-Thr-Phe-Thr-Ser-Asp-Tyr-Ser-Lys-Tyr-Le u-Asp-Glu-Lys-Ala-Ala-Lys(PEG2-PEG2-γGlu-CO(CH2)14C3)-G lu-Phe-Ile-Glu-Trp-Leu-Leu-Arg-Gly-Gly-Gly-Pro-Ser-Ser-Gly- Ala-Pro-Pro-Pro-Ser-NH2 |
| **11** | His-(D-Ser)-Gln-Gly-Thr-Phe-Thr-Ser-Asp-Tyr-Ser-Lys-Tyr-Le u-Asp-Glu-Lys-Ala-Ala-Lys(PEG2-PEG2-γGlu-CO(CH2)16CH3)- Glu-Phe-Ile-Glu-Trp-Leu-Leu-Arg-Gly-Gly-Gly-Pro-Ser-Ser-Gl y-Ala-Pro-Pro-Pro-Ser-NH2 |
| **12** | His-(D-Ser)-Gln-Gly-Thr-Phe-Thr-Ser-Asp-Tyr-Ser-Lys-Tyr-Le u-Asp-Glu-Lys-Ala-Ala-Lys(PEG2-PEG2-γGlu-CO(CH2)16CO2H )-Glu-Phe-Ile-Glu-Trp-Leu-Leu-Asn-Gly-Gly-Gly-Pro-Ser-Ser- Gly-Ala-Pro-Pro-Pro-Ser-NH2 |
| **13** | His-(D-Ser)-Gln-Gly-Thr-Phe-Thr-Ser-Asp-Tyr-Ser-Lys-Tyr-Le |
| | u-Asp-Glu-Lys-Ala-Ala-Lys(PEG₂-PEG₂-γGlu-CO(CH₂)₁₄CO₂H )-Glu-Phe-Ile-Glu-Trp-Leu-Leu-Asn-Gly-Gly-Gly-Pro-Ser-Ser- Gly-Ala-Pro-Pro-Pro-Ser-NH₂ |
| **14** | His-(D-Ser)-Gln-Gly-Thr-Phe-Thr-Ser-Asp-Tyr-Ser-Lys-Tyr-Le u-Asp-Glu-Lys-Ala-Ala-Lys(PEG₂-PEG₂-γGlu-CO(CH₂)₁₆CH₃)- Glu-Phe-Ile-Glu-Trp-Leu-Leu-Asn-Gly-Gly-Gly-Pro-Ser-Ser-Gl y-Ala-Pro-Pro-Pro-Ser-NH₂ |
| **15** | His-(D-Ser)-Gln-Gly-Thr-Phe-Thr-Ser-Asp-Tyr-Ser-Lys-Tyr-Le u-Asp-Glu-Lys-Ala-Ala-Lys(PEG2-PEG2-γGlu-CO(CH2)14CH3)- Glu-Phe-Ile-Glu-Trp-Leu-Leu-Asn-Gly-Gly-Gly-Pro-Ser-Ser-Gl y-Ala-Pro-Pro-Pro-Ser-NH2 |
| **16** | His-(D-Ser)-Gln-Gly-Thr-Phe-Thr-Ser-Asp-Tyr-Ser-Lys-Tyr-Le u-Asp-Lys-Lys(PEG₂-PEG₂-γGlu-CO(CH₂)₁₄CH₃)-Arg-Ala-Gln- Asp-Phe-Val-Gln-Trp-Leu-Met-Asn-Thr-Gly-Gly-Pro-Ser-Ser- Gly-Ala-Pro-Pro-Pro-Ser-NH₂ |
| **17** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)16CO2H)-Arg-Arg-Ala-Gl n-Asp-Phe-Val-Gln-Trp-Leu-Met-Asn-Thr-Gly-Gly-Pro-Ser-Ser -Gly-Ala-Pro-Pro-Pro-Ser-NH2 |
| **18** | His-(D-Ser)-Gln-Gly-Thr-Phe-Thr-Ser-Asp-Tyr-Ser-Lys-Tyr-Le u-Asp-Lys(PEG2-PEG2-CO(CH2)16CO2H)-Arg-Arg-Ala-Gln-As p-Phe-Val-Gln-Trp-Leu-Met-Asn-Thr-Gly-Gly-Pro-Ser-Ser-Gly -Ala-Pro-Pro-Pro-Ser-NH2 |
| **19** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)16CH3)-Arg-Arg-Ala-Gln -Asp-Phe-Val-Gln-Trp-Leu-Met-Asn-Thr-Gly-Gly-Pro-Ser-Ser- Gly-Ala-Pro-Pro-Pro-Ser-NH2 |
| **20** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)14CH3)-Arg-Arg-Ala-Gln -Asp-Phe-Val-Gln-Trp-Leu-Nle-Asn-Thr-Gly-Gly-Pro-Ser-Ser- Gly-Ala-Pro-Pro-Pro-Ser-NH2 |
| **21** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)16CO2H)-Arg-Arg-Ala-Gl n-Asp-Phe-Val-Gln-Trp-Leu-Nle-Asn-Thr-Gly-Gly-Pro-Ser -Ser-Gly-Ala-Pro-Pro-Pro-Ser-NH2 |
| **22** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)16CO2H)-Arg-Arg-Ala-Gl n-Asp-Phe-Val-Gln-Trp-Leu-Leu-Asn-Thr-Gly-Gly-Pro-Ser -Ser-Gly-Ala-Pro-Pro-Pro-Ser-NH2 |
| **23** | His-(D-Ser)-Gln-Gly-Thr-Phe-Thr-Ser-Asp-Tyr-Ser-Lys-Tyr-Le u-Asp-Lys(PEG2-PEG2-γGlu-CO(CH2)14CH3)-Arg-Arg-Ala-Gln -Asp-Phe-Val-Gln-Trp-Leu-Leu-Asn-Thr-Gly-Gly-Pro-Ser-Ser- Gly-Ala-Pro-Pro-Pro-Ser-NH2 |
| **24** | His-(D-Ser)-Gln-Gly-Thr-Phe-Thr-Ser-Asp-Tyr-Ser-Lys-Tyr-Le u-Asp-Lys(PEG2-PEG2-CO(CH2)16CO2H)-Arg-Arg-Ala-Gln-As p-Phe-Val-Gln-Trp-Leu-Nle-Asn-Thr-Gly-Gly-Pro-Ser-Ser-Gly- Ala-Pro-Pro-Pro-Ser-NH2 |
| **25** | His-(D-Ser)-Gln-Gly-Thr-Phe-Thr-Ser-Asp-Tyr-Ser-Lys-Tyr-Le u-Asp-Lys(PEG2-PEG2-CO(CH2)14CH3)-Arg-Arg-Ala-Gln-Asp- Phe-Val-Gln-Trp-Leu-Leu-Asn-Thr-Gly-Gly-Pro-Ser-Ser-Gly-A la-Pro-Pro-Pro-Ser-NH2 |

### Example 2: In vitro inhibition effect of GLP-1R/GCGR dual target agonist polypeptide derivatives on hepatic fibrosis

Hepatic stellate cell strain LX-2 (from China Center for Type Culture Collection, Shanghai) was selected to study and observe the effects of polypeptide compounds 1-25 on the expression level of LX-2 cell activation marker α-smooth muscle actin (α-SMA). The control group was given the same volume of Dulbecco's modified Eagle medium (DMEM) (from Gibco, United States), and β-actin (from TransGen Biotech) as the endogenous reference, wherein, α-SMA is a symbolic feature that distinguishes myoblast cells from myofibroblasts.

Hepatic stellate cell LX-2 was laid on 35 mm cell culture plate, cultured with DMEM (high glucose)+10% FBS+1% double-antibody culture medium (Thermo Fisher) , placed overnight when the cells grew to 70% convergence at 37°C under the condition of 5% CO₂ without serum, and treated with the above GLP-1R/GCGR dual target agonist polypeptide derivatives (namely, polypeptide compounds 1-25) (dissolved in PBS) for 48 hours from the next morning to extract cell protein, and then Western Blot was performed with β-actin as endogenous reference to analyze the expression levels of α-SMA and β-actin by gray-scale analysis using Image J 1.50i. PBS with the same volume as that in experimental group was added in negative control.

The above polypeptide compounds 1-25 with a concentration of 10.0 µM were added, showing that they could reduce the expression of α-SMA at the concentration of 10.0 µM; and the negative control had no effect on the expression of α-SMA (referring to Table 2).

Table 2 shows the experimental results of in vitro inhibition on hepatic fibrosis with polypeptide compounds 1-25 with a concentration of 10.0 µM of the present invention and Liraglutide standard (from GL Biochem (Shanghai) Ltd., purity > 98%, Liraglutide Acetate Cas No.: 204656-20-2). The integrated gray scale of α-SMA/β-actin in the negative control group was considered to be 100% to analyze the in vitro inhibitory activity on hepatic fibrosis by tested polypeptides. **Table 2. Effects of polypeptide compounds 1-25 on relative expression of cell**

**activation marker α-SMA of hepatic stellate cell strain LX-2.**

| **Polypeptide (SEQ ID NO:)** | Concentration (µM) | Relative expression of α-SMA (α-SMA/β-actin, %) |
|---|---|---|
| **Negative control** | 0 | 100 |
| **1** | 10.0 | 57.59 |
| **2** | 10.0 | 34.65 |
| **3** | 10.0 | 45.28 |
| **4** | 10.0 | 40.19 |
| **5** | 10.0 | 37.04 |
| **6** | 10.0 | 42.34 |
| **7** | 10.0 | 46.53 |
| **8** | 10.0 | 39.92 |
| **9** | 10.0 | 40.89 |
| **10** | 10.0 | 46.35 |
| **11** | 10.0 | 35.78 |
| **12** | 10.0 | 41.99 |
| **13** | 10.0 | 40.46 |
| **14** | 10.0 | 47.85 |
| **15** | 10.0 | 37.25 |
| **16** | 10.0 | 40.86 |
| **17** | 10.0 | 38.78 |
| **18** | 10.0 | 45.95 |
| **19** | 10.0 | 44.78 |
| **20** | 10.0 | 32.58 |
| **21** | 10.0 | 43.39 |
| **22** | 10.0 | 48.76 |
| **23** | 10.0 | 36.15 |
| **24** | 10.0 | 52.65 |
| **25** | 10.0 | 39.17 |
| **Liraglutide** | 10.0 | 73.68 |

It can be seen from Table 2 that the GLP-1R/GCGR dual target agonist polypeptide compounds 1-25 of the present invention indicate the expression of more excellent in vitro inhibiting hepatic stellate cell LX-2 cell activation marker α-SMA compared with the GLP-1 analogue Liraglutide. Wherein, polypeptide compounds 2, 5, 11, 15, 20, 23 and 25 were selected for further in vivo zoological evaluation.

### Example 3: Improvement effect of GLP-1R/GCGR dual target agonist polypeptide derivatives on fibrosis of HBV-infected humanized mouse liver (hepatitis virus-infected humanized mouse fibrosis model)

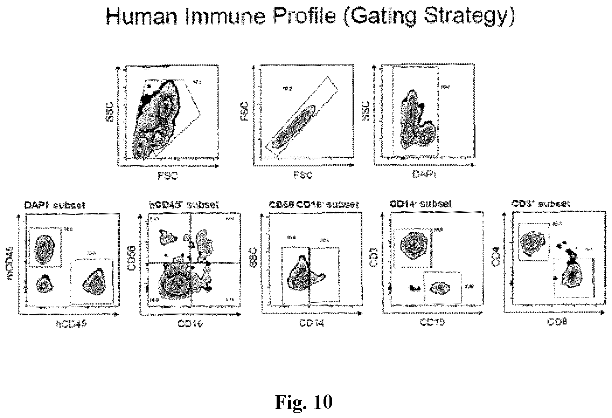

In order to study the effect of the polypeptide compounds of the present invention in hepatic fibrosis associated with viral hepatitis, the polypeptide compounds of the present invention were used for hepatic fibrosis of HBV-infected humanized mouse. HBV does not infect natural mice, so the mice should be humanized before infection with HBV to develop hepatic fibrosis associated with viral hepatitis. The specific steps are shown as follows:
**Humanization of mice**
   (1) Forty newborn NOD-scid IL-2Ry-/- (NIKO) mice were bred.
   (2) These mice were injected with human stem cells for humanization (2 weeks).
   (3) Referring to Fig. 10, blood samples were taken to determine the humanization level by flow cytometry. All the mice used in the experiment expressed humanization according to in vivo concentrations of CD4 and CD8.
**Fibrosis of humanized mouse liver**
   (1) The mice were divided into 8 groups, fed with high-fat diet and infected with HBV (1 week).
   (2) The development of fibrosis in mice was monitored (8-10 weeks).
**Treatment with polypeptide compounds**
   (1) The polypeptide compounds 2, 5, 11, 15, 20, 23 and 25 were selected, which were stored at -20 °C.
   (2) The above 40 mice were divided into 8 groups for injection treatment, with grouping and injection administration as follows: blank model control group (CTR), physiological saline, intraperitoneal injection, n=5.
      1) Administration group, polypeptide compound 2, 40 ug/Kg, intraperitoneal injection, n=5.
      2) Administration group, polypeptide compound 5, 40 ug/Kg, intraperitoneal injection, n=5.
      3) Administration group, polypeptide compound 11, 40 ug/Kg, intraperitoneal injection, n=5.
      4) Administration group, polypeptide compound 15, 40 ug/Kg, intraperitoneal injection, n=5.
      5) Administration group, polypeptide compound 20, 40 ug/Kg, intraperitoneal injection, n=5.
      6) Administration group, polypeptide compound 23, 40 ug/Kg, intraperitoneal injection, n=5.
      7) Administration group, polypeptide compound 25, 40 ug/Kg, intraperitoneal injection, n=5.
   They were injected once every other day for 3 weeks.
(3) After 3 weeks of administration, the mice were sacrificed to take samples for subsequent bioassay experiments. The serum samples from the mice were used for ALT and human IFNy tests. Paraffin sections were prepared from partial liver tissues of the mice and stained with hematoxylin-eosin (H&E), fluorogold/Sirius red (FG/SR) and immunohistochemical fluorescence (α-SMA), and statistics were made with Image-pro plus, Graph pad and similar software.

### Efficacy evaluation and result analysis

In the HBV induced hepatic fibrosis model, its characteristics are: inflammatory cell infiltration around the central vein area, hydropic degeneration of hepatocytes, and increased accumulation of collagen fibers in the portal area and the hepatic interlobular septum. After 3 weeks of administration, the mice were sacrificed to take samples. Blood was taken from the retrobulbar vein for detection of serological indicators, and liver tissues were taken for pathological analysis.

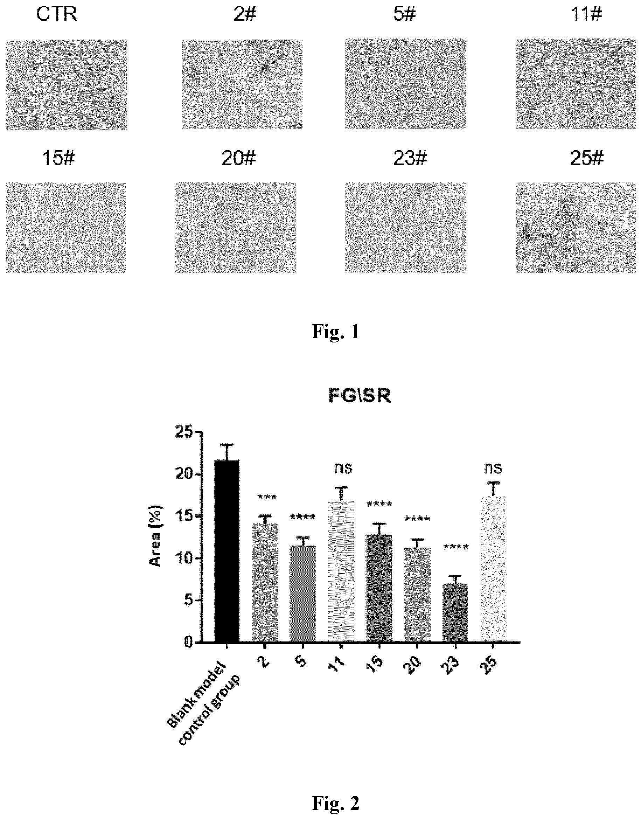

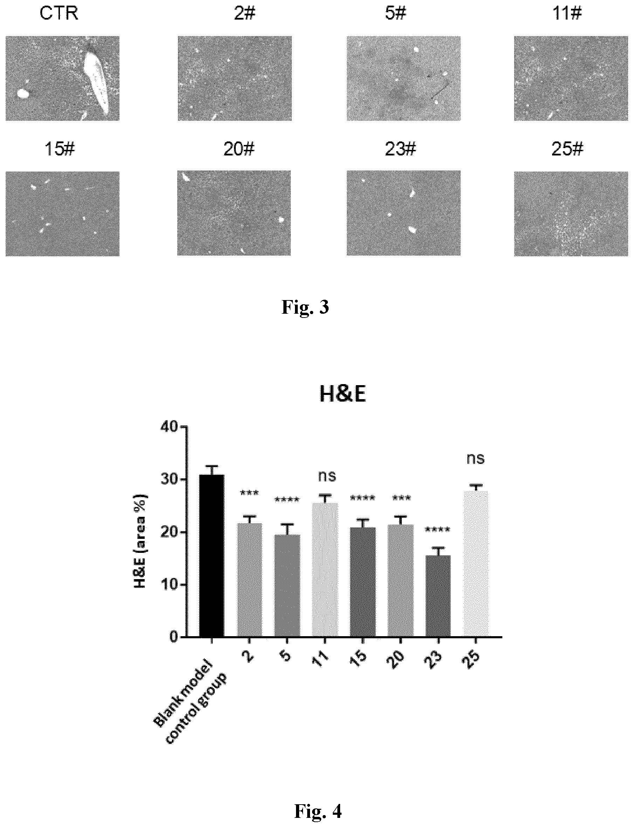

Referring to the FG/SR staining pathological section of liver in Fig. 1, the H&E staining pathological section in Fig. 3, the statistical histogram of FG/SR staining pathological section of liver in Fig. 2 and the statistical histogram of H&E staining pathological section of liver in Fig. 4, compared with the control group without administration, the administration groups of the polypeptide compounds 2, 5, 11, 15, 20, 23 and 25 significantly inhibited the accumulation of collagen fibers and decreased the hepatic fibrosis and inflammation level in mice. It shows that the polypeptide compounds 2, 5, 11, 15, 20, 23 and 25 can effectively inhibit the accumulation of ECM and can treat and improve hepatic fibrosis.

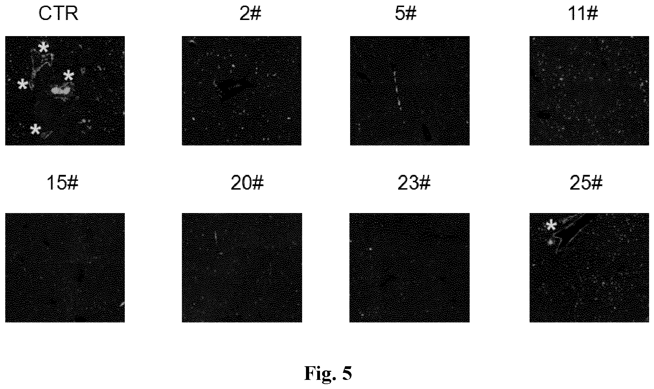

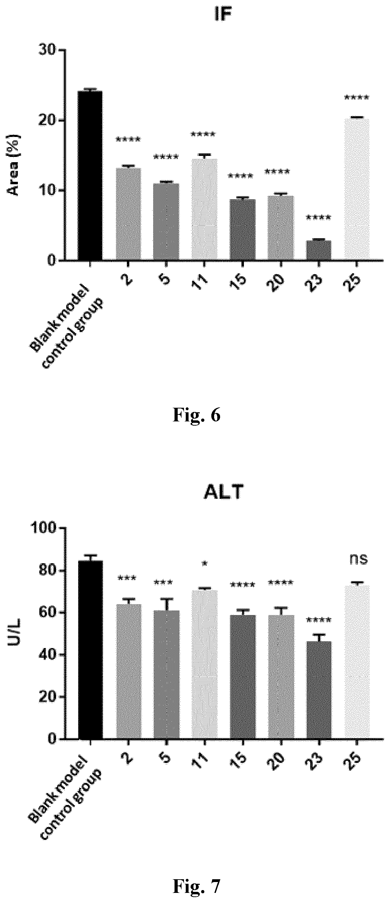

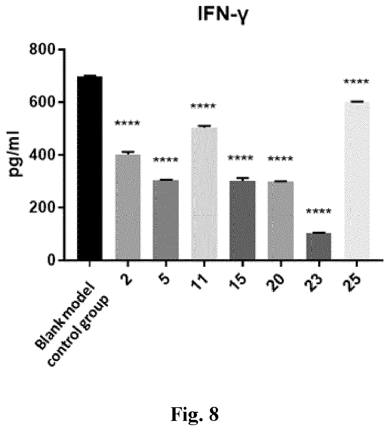

Referring to the α-SMA IF diagram of liver in Fig. 5 and the α-SMA IF statistical histogram of liver in Fig. 6, there is shown the expression of α-SMA in liver tissues, and α-SMA protein is expressed positively around blood vessels by IF staining. The α-SMA was significantly inhibited in the administration groups of the polypeptide compounds 2, 5, 11, 15, 20, 23 and 25. Referring to Fig. 7, there is shown the statistical histogram of serum ALT in the mice. After treatment with the above polypeptide compounds 2, 5, 11, 15, 20, 23 and 25, the expression of ALT in the serum of mice from the model administration group decreased from that before administration. Referring to Fig. 8, there is shown the statistical histogram of human IFN-y expression in serum of the mice. IFN-y is a proinflammatory cytokine produced by Th1 cells and a marker of inflammation. After administration with the above compounds, the serum IFN-y expression in the mice from the treatment groups decreased, the inflammation was remarkably improved, indicating a good therapeutic efficacy.

## Claims

1. Use of GLP-1R/GCGR dual target agonist polypeptide derivatives in preparing drugs for preventing or treating hepatic fibrosis diseases associated with viral hepatitis, the polypeptide derivatives comprising a parent peptide represented by the following amino acid sequence: 
His-Xaa2-Gln-Gly-Thr-Phe-Thr-Ser-Asp-Xaa10-Ser-Lys-Xaa13-Leu-Asp-Xaa
16-Xaa17-Xaa18-Ala-Xaa20-Xaa21-Phe-Xaa23-Xaa24-Trp-Leu-Xaa27-Xaa28-Xa
a29-Gly-Gly-Pro-Ser-Ser-Gly-Ala-Pro-Pro-Pro-Ser-COR1
 wherein, R₁ = -NH₂;
Xaa2 = Aib or D-Ser;
Xaa10 = Lys or Tyr;
Xaa13 = Lys or Tyr;
Xaa16 = Glu or Lys;
Xaa17 = Lys or Arg;
Xaa18 = Arg or Ala;
Xaa20 = His, Gln or Lys;
Xaa21 = Asp or Glu;
Xaa23 = IIe or Val;
Xaa24 = Glu or Gin;
Xaa27 = Met, Leu or Nle;
Xaa28 = Asn, Asp or Arg;
Xaa29 = Gly or Thr.

2. The use according to claim 1, wherein at least one of Xaa10, Xaa16, Xaa17 or Xaa20 is Lys, the side chain of the at least one Lys or the Lys at position 12 of the amino acid sequence is attached to a lipophilic substituent in such a way that a carboxyl group of the lipophilic substituent forms an amide bond with an amino group of a bridging group, the bridging group is attached to the parent peptide by means of a carboxyl group of the bridging group which forms an amide bond with a N-terminal residue of the Lys of the parent peptide, wherein the bridging group is Glu, Asp and/or (PEG)ₘ, and m is an integer of 2 to 10; and the lipophilic substituent is an acyl group selected from a group consisting of CH₃(CH₂)ₙCO- and HOOC(CH₂)ₙCO-, wherein n is an integer of 10 to 24.

3. The use according to claim 2, wherein the bridging group is Glu-(PEG)ₘ or Asp-(PEG)ₘ or (PEG)ₘ.

4. The use according to claim 2, wherein a molecular bridge is formed by means of the bridging group between the side chains of amino acid residue pairs 12 and 16, 16 and 20, 17 and 21, or 20 and 24 in the amino acid sequence.

5. The use according to claim 2, wherein the Lys attached to the lipophilic substituent is replaced with HomoLys, Orn, Dap or Dab.

6. The use according to any one of claims 2-5, wherein when the position 10, 12, 16, 17, or 20 of the amino acid sequence is Lys, the side chain of the Lys is attached to the lipophilic substituent and the bridging group in one of the following structures:
Lys(PEG₂-PEG₂-CO(CH₂)₁₄CH₃): 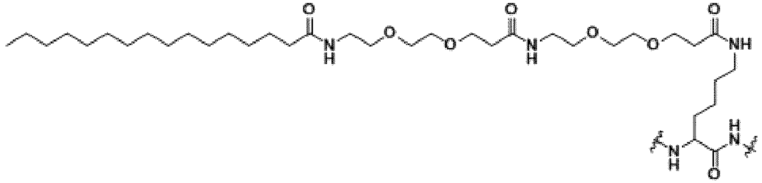
Lys(PEG₂-PEG₂-γGlu-CO(CH₂)₁₄CH₃): 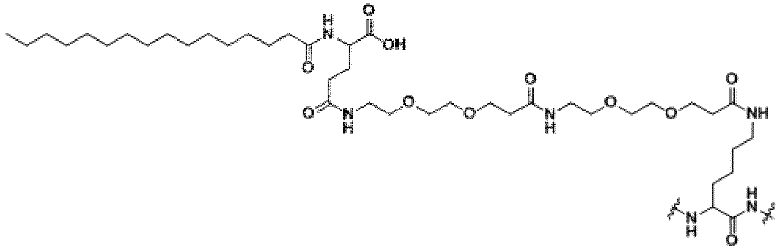
Lys(PEG₂-PEG₂-CO(CH₂)₁₄CO₂H): 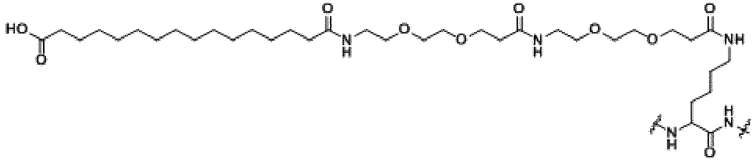
Lys(PEG₂-PEG₂γGlu-CO(CH₂₎₁₄CO₂H): 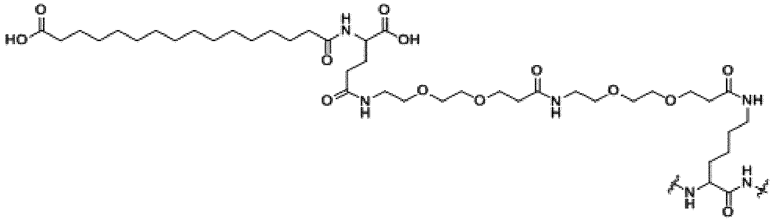
Lys(PEG₂-PEG₂-CO(CH₂)₁₆CO_{2H}): 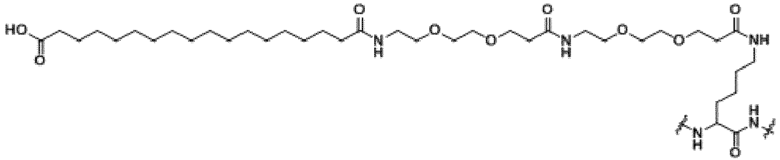
Lys(PEG₂-PEG₂-γGlu-CO(CH₂)₁₆CO₂H): 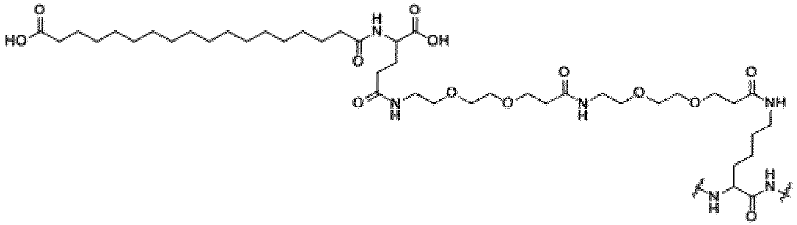
Lys(PEG₂-PEG₂-CO(CH₂)₁₆CH₃): 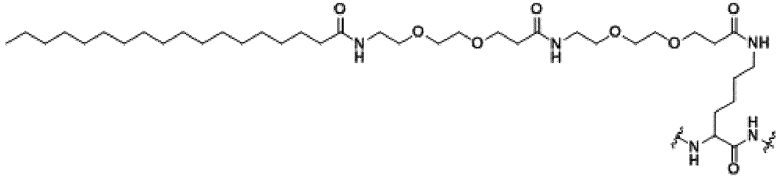
Lys(PEG₂-PEG₂-γGlu-CO(CH₂)₁₆CH₃):

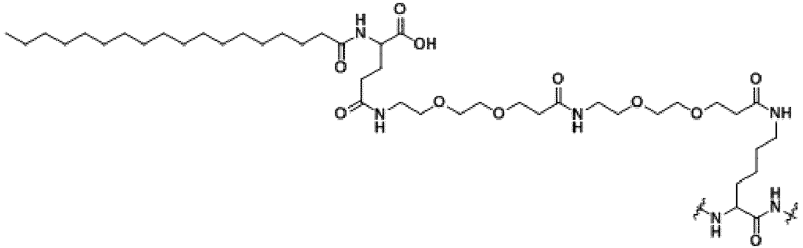

7. The use according to claim 1, wherein the amino acid sequence of the parent peptide is selected from SEQ ID NO:1, SEQ ID NO:2, SEQ ID NO:3, SEQ ID NON, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24 and SEQ ID NO:25.

8. The use according to claim 1, wherein the GLP-1R/GCGR dual target agonist polypeptide derivatives are used for preparing drugs for preventing, directly treating or indirectly treating diseases caused by or featured by the hepatic fibrosis course associated with the viral hepatitis.

9. The use according to claim 1, wherein the hepatic fibrosis diseases associated with the viral hepatitis comprise the HBV-induced and HCV-induced hepatic fibrosis.

10. A composition comprising the GLP-1R/GCGR dual target agonist polypeptide derivatives according to any one of claims 1 to 8 and at least one pharmaceutically acceptable pharmaceutical carrier.

11. The composition according to claim 10, wherein the composition is in at least one dosage form of tablet, capsule, sugar-coated tablet, granule, oral liquid, syrup, ointment and paste applied on skin surface, aerosol, nasal spray and sterile solutions for injection.
